Europäisches Patentamt

European Patent Office

Office européen des brevets

(11) Publication number: **0 342 795**
**A2**

## EUROPEAN PATENT APPLICATION

(21) Application number: 89303830.7

(51) Int. Cl.⁴: **A61K 31/20**

(22) Date of filing: 18.04.89

The title of the invention has been amended (Guidelines for Examination in the EPO, A-III, 7.3).

(30) Priority: 18.04.88 JP 94789/88

(43) Date of publication of application:
23.11.89 Bulletin 89/47

(84) Designated Contracting States:
DE FR GB

(71) Applicant: TAIYO GYOGYO KABUSHIKI KAISHA
No. 1-2, 1-chome Ohtemachi
Chiyoda-ku Tokyo(JP)

(72) Inventor: Kimura, Shoji
2-9, 3-Chome Tsukishima Chuo-ku
Tokyo(JP)
Inventor: Fujimoto, Kenshiro
1-205, Daiichichiku Kawauchi zyutaku
Kawauchi mubanchi Sendai-Shi
Miyagi-Ken(JP)
Inventor: Nishikawa, Masazumi
2-9, 3-Chome Tsukishima Chuo-ku
Tokyo(JP)
Inventor: Maruyama, Kazuaki
2-9, 3-Chome Tsukishima Chuo-ku
Tokyo(JP)
Inventor: Nonaka, Michio
2-9, 3-Chome Tsukishima Chuo-ku
Tokyo(JP)

(74) Representative: Pett, Christopher Phineas et al
Frank B. Dehn & Co. European Patent
Attorneys Imperial House 15-19 Kingsway
London WC2B 6UZ(GB)

(54) Use of docosahexaenic acid for improving brain function.

(57) There is provided a brain function improving composition containing docosahexaenic acid (DHA) or one of its derivatives or precursors as active ingredient and having an effect of improving the brain function and curing brain malfunctions that can be used for medicines and functional foods or appropriately mixed with a carrier, an excipient or a diluent to provide a liquid agent, a powdery agent, a granular agent, an agent in the form of tablets or an agent taking any other desired form. Such a medical agent or a food containing docosahexaenic acid or at least one of its derivatives is, when taken into the human body, can improve the learning ability and memory of the user. Besides, a brain function improving composition according to the invention can effectively prevent and/or cure dementia.

## Brain function improviding composition

## BACKGROUND OF THE INVENTION

This invention relates to a substance effective for improving cerebral function. More particularly it relates to compositions for improving cerebral function, such as for reinforcing learning ability, improving memory, preventing and/or curing dementia and for providing a functional food.

[Prior Art]

Recently massive efforts have been made on research and development of substances and means that can contribute to improvement of the brain function such as learning ability and memory as well as prevention of brain diseases such as dementia. There have been reported a considerable number of achievements in this field of study which can be roughly divided into two categories; an approach of activating the brain function or improving the brain metabolism by accelerating absorbtion of nutrient by the brain and an approach of improving blood circulation in the brain by supplying nourishment and oxygen to the brain which are indispensable for good blood circulation in the brain. Medicines and curative methods effective for both approaches have also been developed. A remarkable achievement in this field is the discovery that cerebral malfunctions (dementia) are categorized into two types; Alzheimer's disease type dementia which is caused by malfunction of the brain nervous system and blood vessel type dementia which is caused by cerebral angiopathy. Researches for medicines and curative methods for dementia of both types have been under way for sometime.

However, it should be noted that any researches in the area have not reached a stage where a major breakthrough is seen in theoretical as well as practical sense of the word. Only few cheimical agents or medicines have ever been reported to date to show any definitive therapeutic effects.

Some of the few agents that have been proved to be effective for memory improvement and curative medicines for senile dementia include ① a memory improving agent containing as its effective ingredient 2-(7-indenyl oxymethyl) morpholine or a salt of the compound obtained therefrom by adding thereto an acid (Japanese Patent Disclosure No. 56-123915), ② an agent for curing Alzheimer's disease comprising a salt of diferroxamine obtained by adding thereto a pharmacologically permissible acid (Japanese Patent Disclosure No. 58-12123) ③ a memory improving agent containing a dipeptide compound expressed by H-X-Y-OH (where X and Y respectively represent Tyr and Arg or vice versa) (Japanese Patent Disclosure No. 58-170719), ④ a memory defect curing agent containing as its effective ingredient 3,7-dihydro-methyl-1-(t-oxohexyl)-7-propyl-1H-purine-2,6-zion (Japanese Patent Disclosure No. 61-119812) and ⑤ a method of curing senile memory defects applied for PCT international patent (Patent Application No. 61-501564).

As we are in a society where longevity now prevails development of medicines and chemical substances of these types are ardently expected from a medical as well as a social view point.

There is therefor still a need to provide, in view of the increasing demand for medicines and effective chemical substances of the above described types, a functional food which improves brain function in terms of learning ability and memory and can prevent and/or cure senile dementia.

We have now found, in the course of study of physiological and pharmaceutical effects of decosohexaenic acid, a compound which is contained in significant amounts in the oil obtained from herrings and sardines, that this substance or its derivatives can be effectively used as medicines and nourishments for improvement of the brain function. A series of animal experiments has shown that this substance and its derivatives have an effect of improving learning ability and memory as well as prevention and cure of dementia and as these sustances are found as ingredients of natural foods and therefore of proven safety.

## SUMMARY OF THE INVENTION

Docosahexaenic acid (hereinafter referred to as DHA) which constitutes the principal ingredient of a functional food according to the present invention is a straight chain alkenoic acid expressed by chemical formula $C_{22}H_{32}O_2$, having 22 carbon atoms with cis-double bonds at positions 4, 7, 10, 13, 16 and 19, a molecular weight of 328 and a melting point between -44.5 and -44.1°C. This substance is abundantly found in fish oil such as that of herrings and also exists as phospholide of mammals in the liver and other organs, although it is characteristically found in the cerebrum. Since DHA and eicosapentaenic acid (EPA) have recently been found to have a physiological effect in suppressing hypercholesterolemia by lowering the level of plasma cholesterol and agglutination of blood platelets, DHA, EPA, their esters and triglyceride are attracting attention of researchers because of their preventative and curative effects on thrombus diseases such as myocardinal infarction and cerebral infarction. However, there has not been any report on pathological effects of DHA including improvement of learning ability and memory as well as prevention and cure of dementia.

We have now been able experimentally to demonstrate that DHA or derivatives or precursors thereof have medical effects of improving learning ability and memory as well as of preventing and curing senile dementia, and have succeeded in producing medical agents and foods having these medical effects. The medical effects of DHA will be described herein in detail by referring to experimental data obtained. In the experiments, a number of rats were fed on feeds containing at least a derivative of DHA as its effective ingredient and the rats were tested for improvement of learning ability and momory along with a control group.

In the experiment on improvement of learning ability, the rats, which were of the Wistar strain, were put into Y-maze boxes with feed containing DHA ethylester as an effective ingredient.

More specifically, eighty Wistar strain male rats, equally four weeks old, were divided into four groups of 20 rats, of which three groups were tested on three different feeds and the last group or control group was fed on feed containing no DHA derivatives.

The feeds for the three tested groups and the control group were prepared with the compound ratio as shown in Table 1. The compositions of the fatty acids of the fat contained in the feeds are shown in Table 2, which illustrates that three distinctively different fatty acids were used.

Table 1

| Compound Ratio of the Ingredients of the Feeds | |
| --- | --- |
| casein | 20% |
| minerals | 4% |
| vitamins | 1% |
| cellulose powder | 4% |
| fat | 10% |
| $\alpha$ -corn starch | 61% |

3

Table 2

| Composition of Fatty Acids in Oil and Grease (% by weight) | | | | |
|---|---|---|---|---|
| | control group feed | E test group feed | T test group feed | R test group feed |
| 14 : 0 | | 0. 1 | 4. 6 | 0. 6 |
| 16 : 0 | 11.5 | 4. 6 | 5. 7 | 8. 6 |
| 16 : 1 ( - 7) | | | 9. 8 | 4. 1 |
| 18 : 0 | 0. 9 | 1. 3 | 1. 0 | 15. 1 |
| 18 : 1 ( - 9) | 3. 3 | 19. 9 | 9. 3 | 5. 4 |
| 18 : 2 ( - 6) | 68. 8 | 17. 5 | 5. 0 | 0. 4 |
| 18 : 3 ( - 3) | 14. 1 | | 3. 5 | |
| 20 : 0 | 0. 2 | | | |
| 20 : 1 ( -7) | 0.5 | | 1. 6 | 0. 1 |
| 20 : 2 | 0. 1 | | | |
| 20 : 4 ( - 6) | 0. 3 | | 6. 3 | 12. 0 |
| 20 : 5 ( - 3) | | 10. 9 | 17. 7 | 17. 0 |
| 21 : 1 | | 1. 7 | | |
| 22 : 5 | | 3. 4 | 3. 3 | 0. 7 |
| 22 : 6 ( - 3) | | 37. 2 | 32. 2 | 36. 0 |

* control group feed : feed containing sun flower oil and oliver oil

* E test group feed : feed mixed with DHA containing ethylester as effective ingredient

* T test group feed : feed mixed with DHA containing triglyceride as effective ingredient

* R test group feed : feed mixed with DHA containing phospholipid as effective ingredient

BRIEF DESCRIPTION OF THE DRAWINGS

The present invention will be described in greater detail by way of examples and by referring to the accompanying drawings, in which

Fig. 1 is a graphic illustration of the Y maze learning progress (rate of correct responses) of the rats of Example 1 below for food acquisition through discrimination of light and dark routes;

Fig. 2 is a graphic illustration showing the number of correct responses and that of incorrect responses of the DHA ester group of Example 1;

Fig. 3 is a graphic illustration showing the number of correct responses and that of incorrect responses of the control group of Example 1;

Fig. 4 is a graphic illustration of the Y maze learning progress (rate of correct responses) of the rats of Example 2 below for food acquisition through discrimination of the light and dark routes;

Fig. 5 is a graphic illustration showing the number of correct responses and that of incorrect resposnes of the DHA triglyceride group of Example 2;

Fig. 6 is a graphic illustration showing the number of correct responses and that of lincorrect responses of the control group of Example 2;

Fig. 7 is a graphic illustration of the Y maza learning progress (rate of correct responses ) of the rats of Example 3 below for food acquisition through discrimination of light and dark routes;

Fig. 8 is a graphic illustration showing the number of correct responses and that of incorrect responses of the DHA phospholipid group of Example 3;

Fig. 9 is a graphic illustration showing the number of correct responses and that of incorrect responses of the control group of Example 3; and

Fig. 10 is a graphic illustration of the levels of blood platelet agglutination measured by an automatic blood platelet agglutination measurement apparatus.

## DETAILED DESCRIPTION OF THE INVENTION

<Example 1> Experiment of the Effect of the "DHA Ester group on improvement of learning ability and memory.

A group of 20 male rats of the Wister strain which were four weeks old were grown for another week on the control feed and then fed by the test feed mixed with DHA containing ethylester (E test feed group in Table 2). Thereafter, each of the rats underwent a shaping process in a separate cage for two weeks during which the rat lost its weight by 15%. Hereinafter the group of the rats fed on the E test feed are referred to as "DHA ester group". The experiment was conducted for all the 20 rats five times a day (total of 100 times a day) for 18 days and the test results were recorded.

In the experiment, each of the rats was placed at a starting point of a maze, where the rat started moving in search of feed. Eventually, he came across a point where the route was bifurcated into a lighted route that led to feed and an unlighted route that led to no feed. A feed searching action that had been successfully completed within 30 seconds from the start was defined as "a correct response", while an action that had taken more than 30 seconds before completion or that had not succeeded in reaching the feed was defined as "an incorrect response". The rats could see the light only when they arrived at the fork of the Y maze. The rats were tested for their learning achievement in terms of successfully reaching the feed and for their memory by repeating the test every day during the experiment. The rats of the control group were fed by the control feed containing sun flower oil and olive oil instead of DHA (see the control feed group in Table 2).

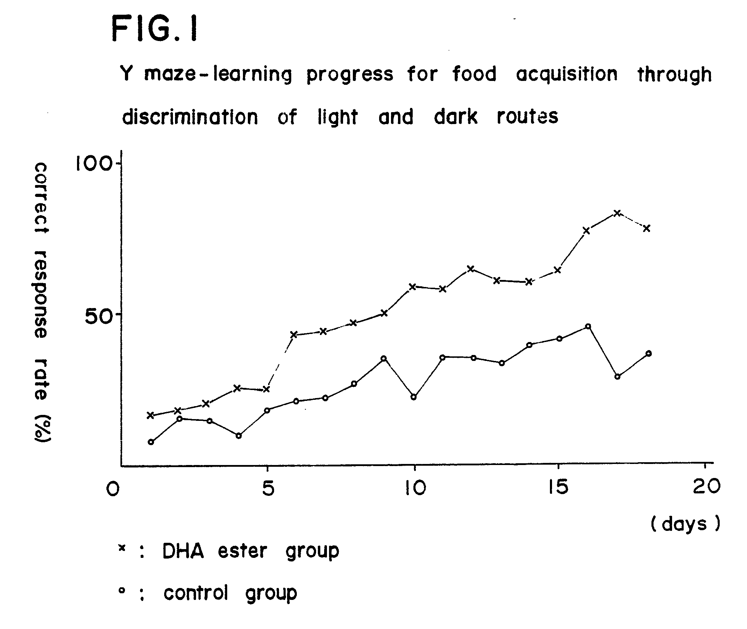

The results of the experiment are illustrated in Figs. 1 through 3.

As shown in Fig. 1, the rate of correct responses of the EHA ester group on the 18th day of the experiment reached as high as 75%, which is significantly higher than that of the control group.

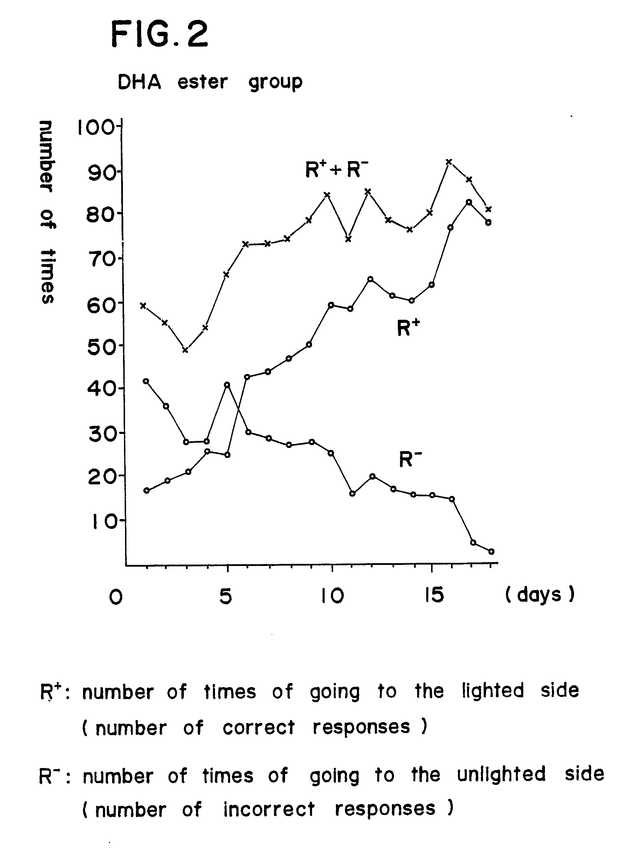

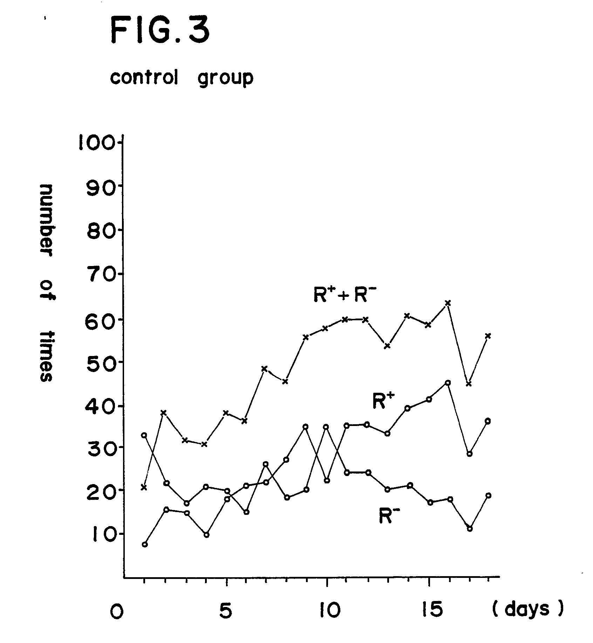

As seen from Fig. 2 showing the learning progress of the DHA ester group and Fig. 3 showing that of the control group, the both groups showed a R- (number of incorrect responses) which is greater than R+ (number of correct responses) in the initial stages of the expepriment because they are nocturnal animals. However, the DHA ester group showed a decline of R- on the 5th day and a considerable increase of R+, while the control group remained practically at a same R- level and the rate of correct responses did not rise significantly. Therefore it is evident that the rats of the DHA ester group learnt quickly that they could reach the feed by following the lighted route and retained the achievement in his memory for many days.

Thus, we have found that the "DHA ester group" feed can improve the learning ability and memory of rats for food acquisition through discrimination of light and dark routes.

<Example 2> Experiment on the Effect of the "DHA Triglyceride Group" Feed on Improvement of Learning Ability and Memory

With a group of rats of the Wister strain, a certain amount of feed prepared by using DHA containing triglyceride (see T test feed group in Table 2) and a Y maze box, the following experiment was conducted.

The 20 male rats of the Wister strain which were four weeks old were fed on the control feed for another week and then fed by the test feed mixed with DHA containing triglyceride (T test feed group in Table 2). Thereafter, each of the rats underwent a shaping process in a separate cage for two weeks during which the rat lost its weight by 15%. Hereinafter, the group of the rats fed on the T test feed are referred to as "DHA triglyceride group".

The experiment was conducted for all the rats five times a day (total of 100 times a days) for 18 days and the results were observed and recorded.

The procedures of the experiment were similar to those of Example 1 above.

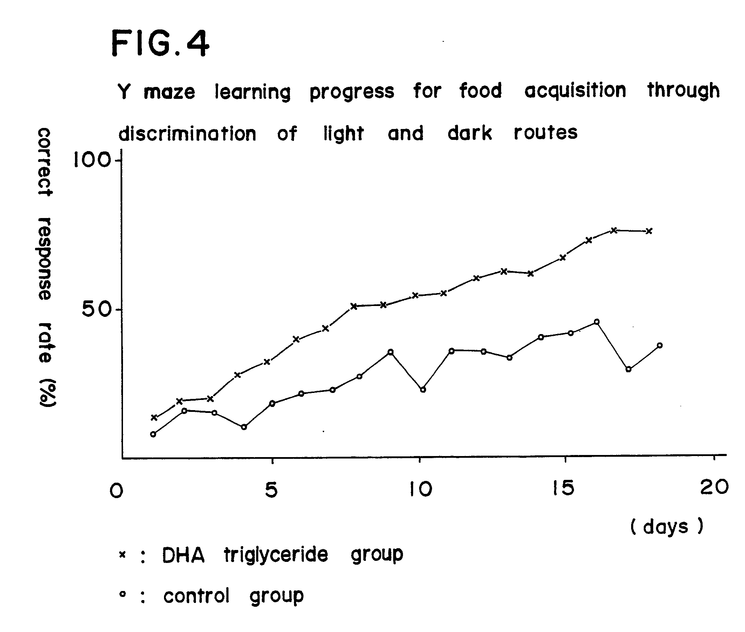

The results of the experiment are illustrated in Figs. 4 through 6.

As shown in Fig. 4, the rate of correct responses of the EHA triglyceride group on the 18th day of the experiment reached 74%, which is significantly higher than that of the control group.

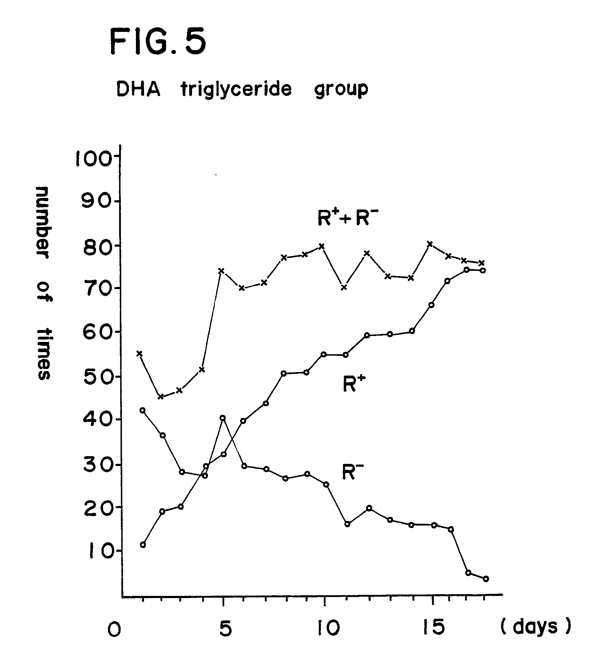

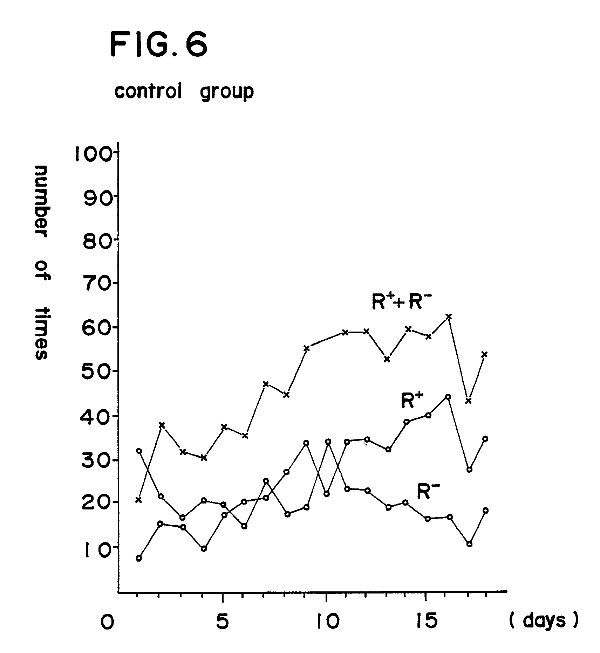

As seen from Figs. 5 and 6, both groups showed a R-(number of incorrect responses) which is greater than R + (number of correct responses ) in the initial stages of the experiment but the "DHA triglyceride group" showed a decline of n and a considerable increase of n on the 6th day, while the control group did not show any decline of R- until the 10th day. Therefore, it is evident that the rats of the DHA triglyceride group learnt quickly that they could reach the feed by following the lighted route and retained the achievement in his memory for many days. Thus, it has been proved that the "DHA triglyceride group" feed can improve the learning ability and memory of rats for food acquisition through discrimination of light and dark routes.

<Example 3> Experiment of the Effect of the "DHA Phospholipid Group" Feed on Improvement of Learning Ability and Memory

A group of 20 male rats of the Wister strain which were four weeks old were grown for another week on the control feed and then fed by the test feed mixed with DHA containing phospholipid. Thereafter, each of the rats underwent a shaping process in a separate cage for two weeks during which the rat lost its weight by 15%. Hereinafter, the group of the rats fed on the feed are referred to as "DHA phospholipid group".

The experiment was conducted for all the 20 rats five times a day (total of 100 times a day) for 18 days and the results were observed and recorded.

The procedures of the experiment were similar to those of Example 1 above.

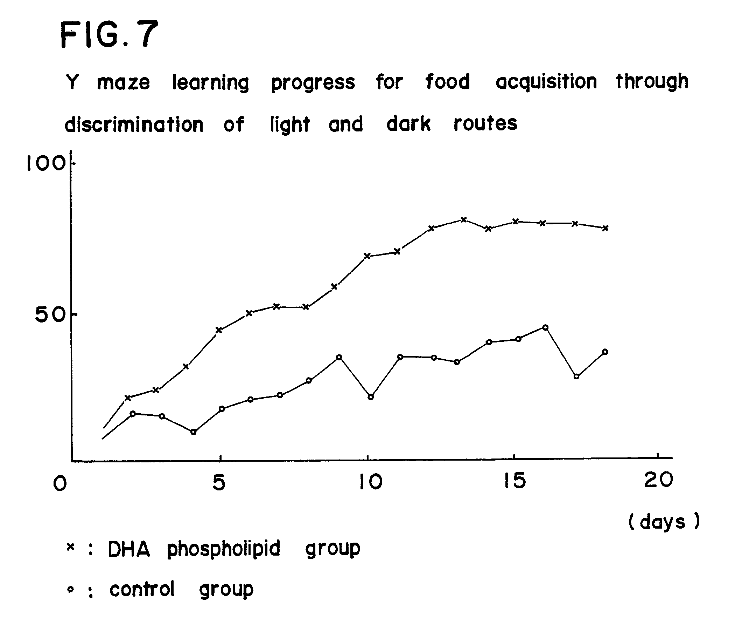

The results of the experiments are illustrated in Figs. 7 through 9.

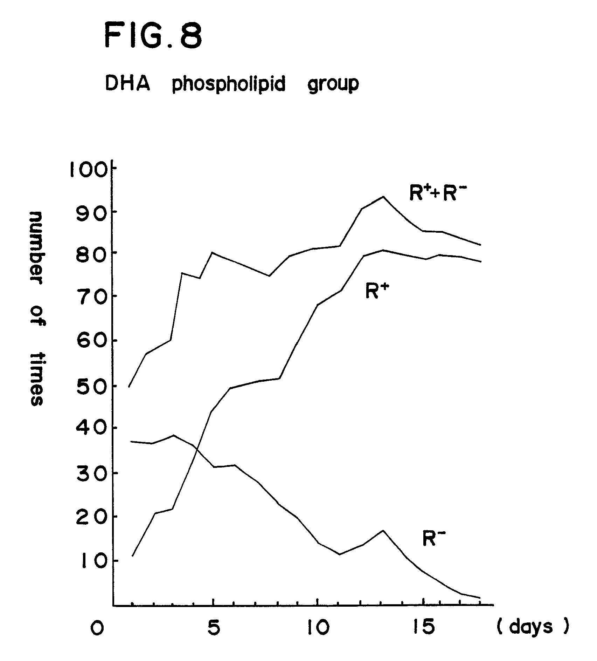

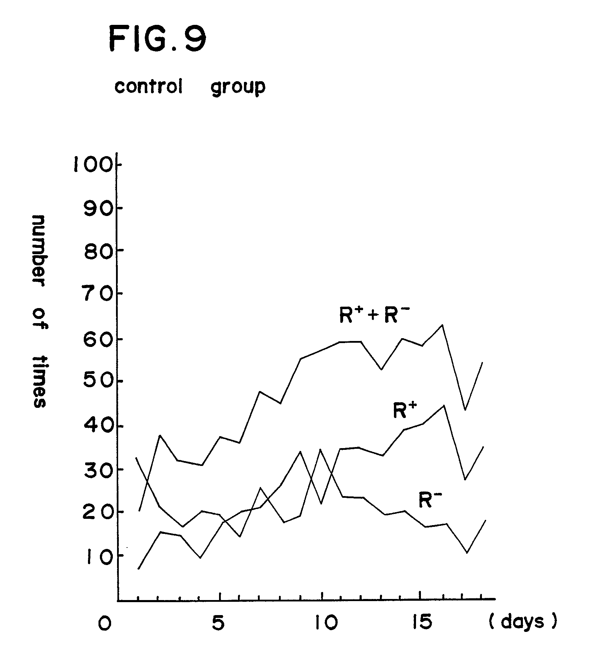

In this experiment, the rate of correct responses of the "DHA phospholipid group" on the 18th day of the experiment reached 73%, which is significantly higher than that of the control group. As seen from Figs. 8 and 9, both groups showed a R- (number of incorrect responses) which is greater than R + (number of correct responses) in the initial stages of the experiment but the DHA phospholipid group showed a decline of R- and a considerable increase of R + on the 4th day, while the control group did not show any decline of R- until the 10th day. Therefore, it is evident that the rats of the DHA phospholipid group learnt quickly that they could reach the feed by following the lighted route and retained the achievement in his memory for many days. Thus, it has been proved that the DHA containing phospholipid can, as in the case of DHA containing triglyceride and DHA containing ethylester, improve the learning ability and memory of rats for food acquisition through discrimination of light and dark routes.

<Example 4> Measurement of Blood Platelet Agglutination

Samples of blood were drawn from ten rats of the DHA ester group that had underwent the experiment on improvement of learning ability for 18 days and the same number of rats of the control group and the samples were subjected to the following measurement.

Nine volume portions of the blood drawn from each of the rats were mixed well with one volume portion of sodium citrate and the mixture was subjected to a separating operation of a centrifugal separator where the separator was revolved at 1,000 r.p.m. for five minutes to obtain platelet rich plasma (hereinafter referred to as P. R. P.) in the supernatant layer which contains blood platelets at a concentration of approximately 20 to 40 $\times$ 10$^4$/m$\ell$. Then the separator was operated at 3.000 r.p.m. for 15 minutes to obtain platelet poor plasma (hereinafter referred to as P. P. P.) in the sedimentary layer. The supernatant liquid was used as blank. The apparatus was Automatic Blood Platelet Agglutination Measurement Apparatus AA-100 (sysmex), a product available from Toa Iyodensi Co. (Toa Medical Electronics Co.) and adenosine-diphosphate (ADP) with a concentration of 9.1$\mu$M was used as trigger for agglutination.

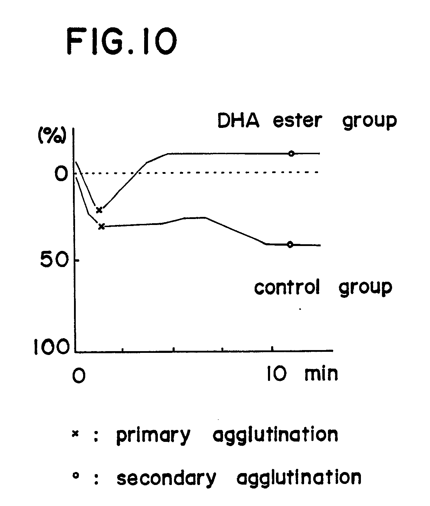

The results of the measurement are shown in Table 3 below and in Fig. 10 of the accompanying drawings.

Table 3

| Platelet Agglutination Measured by an Automatic Platelet Agglutination Measurement Apparatus | | |
|---|---|---|
| | primary agglutination (%) | secondary agglutination (%) |
| control group | 29.3 ± 2.9 | 47.5 ± 6.8 |
| test group | 21.0 ± 1.7 | -9.2 ± 3.3 |

As is apparent from the table above, the rate of the primary agglutination as well as that of the secondary agglutination of the blood of the rats of the DHA ester group were considerably lower than those of the control group and hence it can be said that the DHA ester group feed can effectively prevent thrombus diseases by suppressing agglutination of blood platelets. As dementia is subdivided into two types as described earlier, the Alzheimer's disease type and the blood vessel type, and DHA can effectively prevent cerebral infarction, it is obvious that the substance is effective for prevention and cure of the blood vessel type dementia.

It has also been made evident by a series of experiments conducted separately that DHA containing triglyceride and DHA containing phospholipid are effective ingredients of medical agents having similar preventive and curative effects.

As described above, according to the present invention, a composition containing docosahexaenic acid or one of its derivatives of biological precursors as effective ingredient can, if taken in as food or medical agent, not only effectively improve the brain function in terms of learning ability and memory but also prevent and cure dementia.

Suitable derivatives or precursors of docosahexaenic acid which provide activity in vivo include fatty acids, phospholipids or triglycerides that can be extracted from natural oil and grease, or salts, amides or esters thereof. Esters of the acid may readily be obtained by treatment with a suitable alcohol.

Compositions of the invention will generally comprise the active ingredient appropriately mixed with a suitable carrier, excipient or a diluent to provide a liquid agent, a powdery agent, a granular agent, an agent in the form of tablets, a liquid agent contained in ampules, an encapsulated agent, an agent in the form of suppositories or an agent taking any other desired form. Such an agent may be dosed orally or non-orally. It may be needless to say that the dose of such an agent may readily be determined by consideration of age, body weight, symptom and other factors of the patient.

## Claims

1. A composition for improving cerebral function which comprises docosohexaenic acid or a derivative or precursor thereof in association with a physiologically acceptable carrier diluent or excipient.

2. The use of docosohexaenic acid or a derivative or precursor thereof in the preparation of a medicament for improving cerebral function.

3. A composition or use as claimed in claim 1 or claim 2 wherein said derivative or precursor of docosahexaenic acid is a fatty acid, phospholipid or triglyceride that can be extracted from natural oil and grease.

4. A composition or use as claimed in any one of claims 1, 2 and 3 wherein said derivative or precursor of docosohexaenic acid is a salt, an amide or an ester thereof.

# FIG. I

Y maze-learning progress for food acquisition through discrimination of light and dark routes

correct response rate (%)

(days)

× : DHA ester group

° : control group

# FIG.2

DHA ester group

R⁺: number of times of going to the lighted side ( number of correct responses )

R⁻: number of times of going to the unlighted side ( number of incorrect responses )

FIG.3

control group

# FIG.4

Y maze learning progress for food acquisition through discrimination of light and dark routes

× : DHA triglyceride group

° : control group

FIG.5

DHA triglyceride group

FIG. 6

control group

# FIG. 7

Y maze learning progress for food acquisition through discrimination of light and dark routes

× : DHA phospholipid group

∘ : control group

# FIG.8

## DHA phospholipid group

FIG.9

control group

# FIG.10

DHA ester group

(%)

0

50

control group

100

0          10  min

× : primary  agglutination

° : secondary  agglutination